Europäisches Patentamt

European Patent Office

Office européen des brevets

(11) Publication number: **0 322 393**
**A1**

# EUROPEAN PATENT APPLICATION

(21) Application number: **88850435.4**

(22) Date of filing: **23.12.88**

(51) Int. Cl.⁴: **C 12 P 19/04**

(30) Priority: **23.12.87 US 137221   16.12.88 US 285633**

(43) Date of publication of application:
**28.06.89 Bulletin 89/26**

(84) Designated Contracting States: **DE FR GB SE**

(71) Applicant: **GEORGE WESTON LIMITED**
**1047 Yonge Street**
**Toronto Ontario M4W 2L3  (CA)**

(72) Inventor: **Lawford, Hugh Gibson**
**2383 Edenhurst Drive**
**Mississauga Ontario L5A 2L1  (CA)**

**Kligerman, Anne**
**24 Elm Drive**
**Toronto Ontario M6B 1A3  (CA)**

**Fein, Jared E.**
**1519 Jasmine Crescent**
**Oakville Ontario L6H 3H2  (CA)**

(74) Representative: **Ström, Tore et al**
**Ström & Gulliksson AB Studentgatan 1 P.O. Box 4188**
**S-203 13 Malmö  (SE)**

(54) **New beta-1,3-glucan exopolysaccharide and process for its preparation.**

(57) Novel, improved, water insoluble, gel-forming β-1,3-glucan exopolysaccharide is described having an intrinsic viscosity of at least about 6.0 dL/g, as measured by the procedure described in the disclosure, and improved gel strength and molecular weight qualitites. The novel exopolysaccharide is produced in a process having two phases, a growth phase and a stationary exopolysaccharide biosynthesis phase. In the latter phase, agitation are aeration are controlled to provide mixing sufficient to produce a homogeneous medium and oxygen transfer sufficient for be aerobic biosynthesis without significantly decreasing the intrinsic viscosity of the exopolysaccharide product. Maintaining the dissolved oxygen concentration in the aqueous medium in the stationary phase at a value of at least 2.0 mg $O_2$/L, preferably at least 6.0 mg $O_2$/L, results in increased specific rate of exopolysaccharide product production. The novel products are useful as thickening agents and the like.

FIG. 1

EP 0 322 393 A1

**Description**

## Background of the Invention

This invention relates to novel improved β-1,3-glucan exopolysaccharides of microbial origin and processes for their preparation.

Polysaccharides are of three general origins, namely plant, animal and microbial. Microbial polysaccharides may be structural components of bacterial cell walls or may be synthesized by certain bacteria as extracellular polymers known as microbial exopolysaccharides (EPS). Exemplary microbial polysaccharides include dextrans, xanthan gum, pullulan and curdlan. Many polysaccharides function as hydrophilic colloids, dramatically altering the flow properties of aqueous systems at relatively low concentrations. Microbial polysaccharides can be used to replace plant gums such as cellulose, pectin and agar for such varied purposes as thickening agents, stabilizers, suspending or flocculating agents, and emulsifiers, especially in the food, cosmetic and pharmaceutical industries. Microbial polysaccharides can also be used as moldable materials such as biodegradable films.

A class of water insoluble β-1,3-glucan exopolysaccharide which exhibits unique rheological and irreversible thermo-gelling properties is known to be produced by species of two different genera of bacteria, Alcaligenes and Agrobacterium. The uncharged polymer can be recovered as an insoluble gel upon neutralization of the alkalinized cell-free fermentation broth (see "Curdlan-A Gel Forming Glucan" by T. Harada, Polysaccharides in Food, eds. J.M.V. Blansard & J.R. Mitchell, Butterworth U.K. (1979) pp. 283-300). In particular it is known that a water-insoluble, thermally-gelable β-1,3-glucan exopolysaccharide comprising a linear polymer of glucose residues connected by β-1,3 linkages and being called "curdlan" can be produced by batch cultivation of selected mutant (strain K) of Alcaligenes faecalis var. myxogenes 10C3 (see Harada et al., Agr. Biol. Chem., 30, 196, 1966; Agr. Biol. Chem., 30, 764, 1966; and Arch. Biochem. Biophys., 124, 292, 1968).

U.S. Patents 3,754,925 and 3,822,250 which issued on August 28, 1973 and July 2, 1974, to Kimura et al., describe a uracil-requiring auxotrophic mutant of A. faecalis var.myxogenes 10C3K designated as strain NTK-u (IFO 13140 and ATCC 21680) which produces a curdlan-like exopolysaccharide stated to have different physicochemical characteristics from the curdlan disclosed by Harada et al. Kimura et al. reported that the gel formed by heating an aqueous suspension of the polysaccharide (PS) derived from this strain (termed PS-13140) is not only more stable but is also superior to the one derived from the previously known samples of curdlan. However, comparative data with known curdlan samples was not provided in the patents. Another curdlan-like polymer, referred to herein as PS-13126, was also described by Kimura et al. and was stated to have physicochemical properties substantially the same as PS-13140. Polymer PS-13126 was produced by batch cultivation of a mutant of Agrobacterium radiobacter, namely strain U-19 (IFO 13126 and ATCC 21679). The parent culture from which this mutant was derived is designated as Agrobacterium radiobacter IFO 13127 (also as ATCC 6466).

In the exopolysaccharide production processes described in the above references, polymer synthesis occurred after growth ceased (i.e. during the stationary growth phase). This batch fermentation process was of approximately four days duration with a carbon-substrate to polymer product conversion efficiency of about 50%. It is known that the productivity of a continuous process is markedly superior to that of a batch process. However, production of secondary metabolites, which are synthesized during the stationary-phase of cultivation after the cessation of growth, is not effectively conducted in single-stage continuous flow systems where the culture is growing. Continuous culture of curdlan-like exopolysaccharide produced by variants of A. faecalis var. myxogenes has been described (see Phillips et al., Can. J. Microbiol., 29, 1331, 1983; Lawford et al., Biotech. Letts., 4, 689, 1982). A two-stage or two-phase cascade, continuous fermentation process for the production of curdlan-type exopolysaccharide using stable non-auxotrophic mutants of A. faecalis var. myxogenes ATCC 21680 (designated as ATCC 31749 and ATCC 31750) is the subject of U.S. Patent 4,355,106 issued to H.G. Lawford on October 19, 1982.

To the inventors' knowledge, there is only one commercially available curdlan-like exopolysaccharide. Wako Pure Chemical Industries Ltd., a division of Takeda Chemical Industries, Ltd., of Japan, supply a product, hereinafter referred to as Wako curdlan product, as a fine chemical.

In previous studies by one of the inventors (see H. Lawford et al., Biotechnol. Letters 8, 145, 1986) it was suggested that bioreactor configuration could be altered to improve the concentration of the β-1,3-glucan EPS in the fermentation broth. That paper reported increased concentrations of the polymer in the fermentation broth could be achieved by using a vibrationally mixed vibro-fermentor or by substituting a marine-type propeller for the uppermost two of the three flat-blade turbine impellers in a conventional baffled turbine-agitated bioreactor.

Whereas the present invention relates to the special oxygen requirements of the culture during the exopolymer production phase (stationary-phase) of the overall fermentation process, as will be explained hereinafter, it is recognized that the oxygen requirements of the growing culture are different. It is known that the respiration rate, and hence oxygen demand, of the growing culture (exponential growth-phase) is much greater than that of the non-growing culture; however, providing the concentration of dissolved oxygen is maintained at a level greater than the growth limiting concentration (called the critical dissolved oxygen concentration, $DO_{crit}$), the culture will not become oxygen limited. The value for $DO_{crit}$ is generally quite low being in the range of 0.2 to 0.4 mg $O_2$/L. In practice, aerobic fermentations are conducted at a DO sufficiently high to avoid problems with oxygen limitation, without providing oxygen at a level which is economically excessive. At the same time, it is also recognized that too high a DO concentration can be detrimental or inhibitory to cellular function and such a condition is to be avoided. Heretofore, in exopolysaccharide fermentations, oxygen was supplied conservatively at a rate so as to result in a DO value that was not excessively above $DO_{crit}$, in accordance with predictable oxygen requirements of non-growing (stationary-phase) cultures. Operating ranges of about 2.5 times $DO_{crit}$ (i.e. 0.5 to 1.0 mg $O_2$/L) would be thought to be sufficient.

Summary of the Invention

In their continued studies of microbial exopolysaccharides and their search for non-ionic, high performance viscosifiers and gelling agents, the inventors discovered that the physicochemical properties of an isolated microbial β-1,3-glucan exopolysaccharide could be improved for certain end use applications by controlling the operating conditions of the bioreactor. Specifically, the intrinsic viscosity of the isolated exopolysaccharide, as used as an indicator of improved rheological properties of the exopolysaccharide, was found to remain above about 6.0 dL/g throughout the fermentation (i.e. independent of elapsed fermentation time in the stationary phase) if moderate agitation during the fermentation, was used. The term intrinsic viscosity, as used herein, refers to the intrinsic viscosity of the isolated exopolysaccharide as measured by the procedure described hereinafter. It was found that agitation in the stationary exopolysaccharide biosynthesis phase could be controlled to provide mixing sufficient to produce a homogeneous medium and oxygen transfer sufficient for the aerobic biosynthesis of the exopolysaccharide product without significantly decreasing the intrinsic viscosity of the exopolysaccharide product. This moderate agitation can be achieved by varying the bioreactor design, particularly by controlling the mechanical mixing and/or the gas sparging (oxygen introduction) used in the stationary phase. This moderate agitation technique causes less vigorous mixing than occurs in a conventional, baffled, turbine-agitated bioreactor.

It should be clarified that the term agitation, as used herein, refers to the mixing achieved by the mechanical stirrers in a bioreactor and the mixing achieved by the gas sparging (oxygen introduction). Since the exopolysaccharide biosynthesis phase is aerobic, oxygen, through gas sparging, must be present in an amount sufficient to prevent oxygen from becoming a limiting factor in the biosynthesis. Stated another way, to achieve mass transfer of oxygen, agitation must provide mixing sufficient to transfer oxygen from the gas into the solution so that oxygen necessary for the biosynthesis is supplied. Also, agitation provides sufficient mixing to produce a homogeneous medium, that is to produce a generally uniform distribution of the reactants and products throughout the medium.

The preferred menthods of agitation in the stationary phase, in accordance with this invention, are agitation devices such as propellers or hydrofoil impellers that cause less vigorous mixing than conventional flat-blade turbine impellers and whereby oxygen transfer is effected with the aid of gas dispersion devices independent of the agitator (mixing device) or whereby oxygen transfer is enhanced by operating the bioreactor with overpressure and/or whereby oxygen transfer is enhanced through oxygen enrichment of the gas being sparged into the liquid in the bioreactor.

The β-1,3-glucan exopolysaccharide produced and isolated by the process of this invention is improved over previously known and isolated β-1,3-glucan exopolysaccharides, by having a significantly increased intrinsic viscosity. Other physicochemical properties of the product of this invention were also found to be favourably influenced, namely molecular weight, gel strength, deformation and compressibility. Other physicochemical properties of the product, including IR, U.V. and N.M.R. spectra, specific rotation and solubility were found to be similar to the physicochemical properties of the Wako curdlan product, and are consistent with it being a curdlan-type, β-1,3-glucan exopolysaccharide.

It should also be clarified that references to the physicochemical properties of the exopolysaccharide products of this invention such as intrinsic viscosity, gel strength and molecular weight, refer to the properties of the isolated exopolysaccharide product. As such, these properties are used as indicators of the quality of the product being produced.

The novel and improved β-1,3-glucan exopolysaccharide of the invention is a high molecular weight, non-ionic, thermogelable polymer which exhibits a higher intrinsic viscosity at low concentrations and a superior gel strength when compared to previously known samples of β-1,3-glucan exopolysaccharides. In addition, the gel formed from the novel exopolysaccharide of this invention is more resilient and is superior to gels from previously known curdlan-type β-1, 3-glucan exopolysaccharides.

A major advantage arises from the inventors' discovery that the use of moderate agitation does not significantly decrease the intrinsic viscosity of the isolated exopolysaccharide produced with elapsed fermentation time (EFT). The product of the invention with an intrinsic viscosity consistently greater than 6.0 dL/g, can be recovered at a time late in the fermentation process. This allows for increased product concentrations of the desired product in the fermentation broth with significant improvements in the process economy.

It should be noted that the inventors' discovery with respect to the increased intrinsic viscosity of the isolated β-1,3-glucan exopolysaccharide product is not analogous to other reported findings with respect to changes in the rheological properties of whole fermentation broth with elapsed fermentation time or with bioreactor design (see for example T. Oolman and H. Harvey, "Non-Newtonian Fermentation Systems, CRC Crit. Rev. Biotechnol., 4, 133, 1986; Reuss et al., Germ. Biochem. Eng., June, 233, 1982). Unlike some other exopolysaccharide products, such as pullulan, the β-1,3-glucan exopolysaccharide of this invention is water insoluble, and therefore does not significantly affect the viscosity of the whole fermentation broth. For a soluble polysaccharide such as pullulan, the viscosity of the fermentation broth would be proportional to the concentration of the exopolysaccharide.

The invention provides an improved, water insoluble, gel-forming β-1,3-glucan exopolysaccharide consisting of linearly connected glucose residues attached through β-1,3 linkages and having an intrinsic viscosity, as measured by the procedure described in the disclosure of at least about 6.0 dL/g.

In another aspect of the invention, a process is provided for the production of the above-described novel product. The process comprises (a) cultivating a β-1,3-glucan exopolysaccharide producing strain of microorganism in two phases, a growth phase and a stationary exopolysaccharide biosynthesis phase, the growth phase being conducted in an aerobic, agitated aqueous medium containing assimilable carbon, nitrogen and inorganic salts, the amount of nitrogen being limited so that at the end of the growth phase the medium contains substantially no assimilable nitrogen, at a temperature between about 27 and 33°C and at a pH between about 5 and 8, the stationary phase being conducted in an aerobic agitated aqueous medium containing assimilable carbon, but substantially no assimilable nitrogen, at a temperature between about 27 and 33°C and a pH between about 5 and 8, wherein agitation in the stationary phase provides mixing sufficient to produce a homogeneous medium and oxygen transfer sufficient for the aerobic biosynthesis of the exopolysaccharide product, without significantly decreasing the intrinsic viscosity of the exopolysaccharide product which is produced; (b) solubilizing the exopolysaccharide product so produced, by treatment with alkali; and (c) isolating the exopolysaccharide from the fermentation broth.

The process of the invention may be carried out using known batch or continuous culture processes (Phillips et al., Can. J. Microbiol. 29, 1331, 1983; H.G. Lawford, U.S. Patent 4,355,106 issued October 19, 1982). Production of the novel β-1,3-glucan exopolysaccharide of this invention occurs during the stationary phase of nitrogen-depleted batch cultures. The process is most preferably conducted in a two-stage continuous procedure, similar to that disclosed in U.S. Patent 4,355,106. Controlled agitation and aeration in accordance with the invention, is used in the second non-growth phase of that process.

The preferred β-1,3-glucan exopolysaccharide producing strains of microorganisms used in the process of the invention are of the genera Alcaligenes and Agrobacterium. The strains of the species Alcaligenes faecalis var. myxogenes are most preferred. For continuous processes, the preferred strains of A. faecalis are ATCC 31749 and ATCC 31750. For either batch or continuous culture with Agrobacterium, the preferred culture is ATCC 21679.

In another aspect of this invention, the inventors discovered that, surprisingly, the specific rate of production ($q_p$) of β-1,3-glucan exopolysaccharide could be significantly increased by maintaining the dissolved oxygen concentration (DO) in the stationary phase at a value of at least 2.0 and more preferably 6.0 mg $O_2$/L. When practised in accordance with the moderate agitation techniques of this invention, it was discovered that the specific rate of product production ($q_p$) could be maximized without compromising the quality of the exopolysaccharide produced, as measured by intrinsic viscosity.

## Brief Description of the Drawings

Figure 1 is a schematic flow sheet showing the process of this invention as practised in two stage continuous process using marine-type propeller for agitation in the second stage.

Figure 2 is a graph of the results of fermentation trials, showing the effect of impeller type on the intrinsic viscosity of the isolated salt free β-1,3-glucan exopolysaccharide product with elapsed fermentation time.

Figure 3 is a graph of the results of the fermentation trials, showing the effect of oxygen transfer rate (OTR) on the specific rate of product production ($q_p$).

Figure 4 is a graph of the results of the fermentation trials, showing the effect of dissolved oxygen concentration (DO) on the specific rate of product production ($q_p$).

## Description of the Preferred Embodiment

The novel improved product of this invention is produced by cultivating a β-1,3-glucan exopolysaccharide producing strain of microorganism, preferably cultures of Alcaligenes or Agrobacterium. Batch and continuous culture procedures have been previously described (see Phillips et al., Can. J. Microbiol. 29, 1331, 1983; H.G. Lawford, U.S. Patent 4,355,106). Two phases, a growth phase and a stationary exopolysaccharide biosynthesis phase, are involved. The β-1,3-glucan exopolysaccharide is produced during the stationary-phase of nitrogen-depleted batch cultures as described in the above references. The process is conducted at a temperature between 27°C and 33°C, preferably at 30°C. The growth phase is conducted at an optimum pH between 6.8 and 7.2, preferably at 7.0, whereas the stationary, exopolysaccharide biosynthesis phase, is conducted at an optimum pH between 5.7 and 6.2, preferably at 5.9.

The microorganism is cultivated in an aerobic aqueous medium containing sources of assimilable carbon, nitrogen and inorganic salts. The carbon source can be selected from a broad range of carbohydrate feedstocks, including glucose, sucrose, maltose, lactose, fructose, xylose, hydrolysate of starch, glycerol and organic acids such as succinic and glutamic acid; most preferably glucose and starch hydrolysates are used. Various sources of assimilable nitrogen may be employed, for example, enzymatic digests of protein (tryptones, casein hydrolysate, corn steep liquor, yeast extract, soybean flour) or inorganic nitrogenous compounds, such as ammonium salts of chloride, sulphate or phosphate. The amount of nitrogen included is such that, at the end of the growth phase, the fermentation broth contains substantially no assimilable nitrogen. For this reason, the use of ammonium salts is preferred, because it is possible to formulate a nitrogen-deficient medium with a known amount of assimilable nitrogen, thereby allowing the control of the amount of cell mass in the stationary phase with excess carbon source.

Whether the produce is produced in a batch or continuous process, in accordance with this invention, agitation is controlled in the stationary phase to provide mixing sufficient to produce a homogeneous medium and oxygen transfer sufficient for the aerobic biosynthesis for the production of the exopolysaccharide, without causing a significant decrease in the intrinsic viscosity of isolated product with elapsed fermentation time. Exemplary agitation techniques are described hereinbelow and in the examples. Aeration in accordance with the preferred practice of this invention is described hereinbelow and in the examples.

Exemplary $\beta$-1,3-glucan exopolysaccharide producing strains of microorganisms are listed in Table 1, however, it is understood that other strains of the two genera Alcaligenes and Agrobacterium may be obtained by selective cultivation or mutation or by genetic engineering techniques to provide microorganisms with the desired exopolysaccharide producing properties. For continuous process, the preferred strains of A. faecalis are ATCC 31749 and ATCC 31750. For either batch or continuous processes with Agrobacterium, the preferred culture is ATCC 21679.

TABLE 1

| Curdlan-like EPS Producing Micro-organism | Culture Collection Designation | |
|---|---|---|
| | ATCC | IFO |
| Alcaligenes faecalis var. myxogenes | 21680 | 13140 |
| | 31749 | |
| | 31750 | |
| Agrobacterium radiobacter | 21679 | 13126 |
| | 6466 | 13127 |
| | | 13259 |
| | | 12607 |

Aeration in the stationary, exopolysaccharide biosynthesis phase of this process is preferably conducted so as to maintain a dissolved oxygen concentration (DO) of at least about 2.0 mg $O_2$/L and more preferably at least about 6.0 mg $O_2$/L, in order to increase the specific rate of exopolysaccharide production ($q_p$) above what it would be if the process were conducted at a level predictably sufficient to meet oxygen demands of the culture, this being a concentration of about 1.0 mg $O_2$/L, all other operating conditions being the same, without compromising the quality of the exopolysaccharide product so produced. Maximal $q_p$ is generally attained by maintaining the DO at a value of at least 6.8 mg $O_2$/L.

In order to calculate the DO at which the process is conducted, it is important to understand the relationship between the rate of oxygen transfer from gas to liquid (OTR) and DO. It is generally understood that OTR can be increased by increasing turbulence and shear to reduce the size of gas bubbles, thereby improving mass transfer by increasing the interfacial area between the gas and liquid phases. However, as discovered by the inventors, vigorous mixing as occurs in a conventional baffled turbine-agitated bioreactor results in a diminshed product quality, therefore an alternative means of improving gas dispersion is used. In general, the gas is introduced through, a sparger of porous material such as sintered-glass, ceramics, or a porous metallic substance such as stainless steel. To improve OTR, it must be recognized that, while the mass transfer coefficient for oxygen ($K_La$) is affected by the sparging rate and the viscosity of the liquid as well as by the particular type of agitation/mixing used, dissolved oxygen concentration is affected by the partial pressure of oxygen ($p_g$) in the ventilating gas, the pressure, the temperature and the chemical composition of the liquid.

The oxygen transfer rate is defined by the following relationship:

OTR = $K_La$ (C*-C)

where $K_La$ = oxygen mass transfer coefficient (reciprocal units of time)

C* = dissolved oxygen concentration at saturation

C = observed dissolved oxygen concentration.

The oxygen uptake rate (OUR) or oxygen demand of the culture is represented by the following relationship:

OUR = $q_{O_2}$ [x]

where

$q_{O_2}$ = specific respiration rate (units of mass $O_2$/unit dry biomass/unit time)

[x] = biomass concentration or culture cell density (units dry biomass/unit volume).

In the process of this invention, the OUR can be determined. The cell density (x) of the nitrogen limited culture is fixed by the amount of assimilable nitrogen added to the fermentation medium. The respiration rate ($q_{O_2}$) during the exopolysaccharide production phase or stationary phase is comprised of two components, maintenance respiration and respiration associated with generating the energy (ATP) required for exopolysaccharide biosynthesis. It has been calculated that, if the P/O ratio associated with oxidative phosphorylation were 2.0, then the respiratory demand in terms of maintenance energy requirements represents about 90% of the total respiration rate of the polymer-producing stationary phase culture when the OUR of a stationary phase nitrogen-deficient culture at a cell density of 3g dry wt/L (pH = 5.9, T = 30°) was measured as 8.1 mmoles $O_2$/L·hr (equivalent to 259 mg $O_2$/L·hr) (see Phillips and Lawford, Can. J. Microbiol. 29, 1270, 1983, for the measurement). Therefore, under similar conditions, the OUR of the culture may be estimated by multiplying the specific respiration rate (2.7 mmoles $O_2$/g cell·hr) by the culture cell density.

The OTR is affected by a number of independent variables in the operation of the process, including the type and size of the agitation/mixing device(s), the geometric design of the bioreactor, including the use of internal baffling, the rotational speed of the agitator, the rate of gas sparging and the means by which the gas is introduced into the reactor. For any given bioreactor design, the OTR can be estimated using the standard chemical method of sulphite oxidation using cupric ions as catalyst, in accordance with the procedure described by Cooper et al., Ind. Eng. Chem., 36, 504-509, 1944. This method does not take into account the effect of chemical composition of the fermentation medium on the mass transfer coefficient. Below, the relationship between OTR and agitation speed or air sparging rate is established experimentally for three reactor designs. These relationships make it possible to predict the OTR of a given reactor design under various operating conditions with respect to agitation and aeration.

For a New Brunswick Scientific Multigen F2000 reactor equipped with three Rushton-type flat-blade turbine impellers on a central shaft in a baffled, flat bottom glass reactor, the relationship between OTR (mmoles $O_2$/L·hr) and agitation speed (RPM) at an air sparging rate of 0.33 V/V/M is approximately:

OTR = 99.2 + 0.21 (agitation speed).

For a modified round bottom New Brunswick Scientific Multigen F2000 reactor, equipped with a 7 cm marine type propeller operating at 500 RPM and sparging air through a sintered-glass disk, the relationship is approximately:

OTR = 2.6 + 165.2 (air sparging rate).

Finally, for this same modified New Brunswick Scientific Multigen F2000 reactor operating at a propeller speed of 600 RPM, the relationship is approximately:

OTR = 12.3 + 156.5 (air sparging rate).

The main factors affecting oxygen solubility are partial pressure of oxygen ($p_g$) being sparged through the medium, sometimes referred to as oxygen tension, temperature and solutes in the medium. The saturation concentration of dissolved oxygen is given by the relationship:

$C^* = H\ p_g$

where $p_g$ is the oxygen pressure of the gas phase (atm)

H is Henry's constant (mg/L.atm), which varies with temperature, and

$C^*$ = saturation concentration of oxygen.

At T = 30°C, H = 36.1 mg $O_2$/L·atm, $C^*$ = 7.55 mg $O_2$/L

As will be shown in the examples to follow, for maximal specific rates of β-1,3-glucan exopolysaccharide production ($q_p$) the DO should be greater than about 6.8 mg $O_2$/L, which is 90% of the saturation value of 7.55 mg $O_2$/L for air in water at 30°C (no overpressure).

In the case where air is used as the sparging gas and where the bioreactor is operated without backpressure or overpressure, the value for OTR is preferably about 10 times the value of OUR to maintain a DO of 90% saturation. This relationship is represented as follows:

OTR = 10 (OUR) = 10 [x] $q_{O_2}$.

The value of $q_{O_2}$ was determined to be 86.3 mg $O_2$/g cell·hr for A. faecalis ATCC 31749 in the stationary phase.

The OUR multiple factor can be derived using the formula

$$\text{OUR multiple factor} = \frac{1}{(1 - 6.8/C^*)}$$

It should be noted that when the sparging gas contains more oxygen than is normally present in air (ie 20.9% $O_2$) or if the bioreactor is operated with a backpressure, the required OTR is reduced and this multiple factor is less than 10. For instance, at a backpressure of 0.05 kg/cm² (0.71 psig), the $C^*$ is 7.91 mg $O_2$/L, so the DO of 6.8 mg $O_2$/L equates to only 86% of saturation and the multiple factor of OUR for maximal exopolysaccharide production is 7.13.

If the sparging gas is enriched in respect to oxygen, the OTR requirement is lessened proportionately. If oxygen enriched air at 30% $O_2$ were introduced (without backpressure on the reactor), C* would be 10.83 mg $O_2$/L at 30°C, so a DO of 6.8 would equate to only 63% of saturation. In such circumstances the preferred OTR would be 2.7 times OUR. It should be understood to persons skilled in the art that oxygen enriched air can be produced with membrane oxygenators, usually supplying oxygen enriched air in the concentration range of 25-35% $O_2$. Too high enrichment values are detrimental or inhibitory to cellular function.

To calculate the value of DO as a function of partial pressure of oxygen in the sparging gas and the total pressure, when the overpressure is measured as kg/cm² over 1 atmosphere:

$$DO = H \text{ (at specified T) } p_g + (-0.129 + 34.77 \, p_g)(\text{overpressure}).$$

From the above relationships, it should be possible to calculate the OTR or DO at which to operate given the other operating conditions being employed, in order to achieve an increased or maximal rate of exopolysaccharide production in accordance with this invention.

Based on the above relationships for OTR and OUR, when the supply of oxyge (OTR) is balanced by the oxygen demand of the culture (OUR), then the following relationship holds:

$$K_La \, (C^* - C) = q_{O_2} \, [x]$$

Since in the measurements of oxygen transfer rate by sulphite oxidation $OTR = K_La.C^*$, the equation can be solved for C as follows:

$$C = C^* \, (1 - OUR/OTR)$$

under specific conditions of temperature, pressure and chemical composition of the liquid. Thus, if both OUR and OTR are known, one can calculate a value for C or DO, the observed dissolved oxygen concentration.

While the cultivation of the β-1,3-glucan exopolysaccharide producing strain can take place in a batch or continuous process, this step will now be described in further detail in accordance with a preferred two-stage cascade continuous process. Greater details for such a process can be found in U.S. Patent 4,355,106, however the second and stationary phase of this process is modified in accordance with the present invention in order to produce the novel exopolysaccharide product of high intrinsic viscosity.

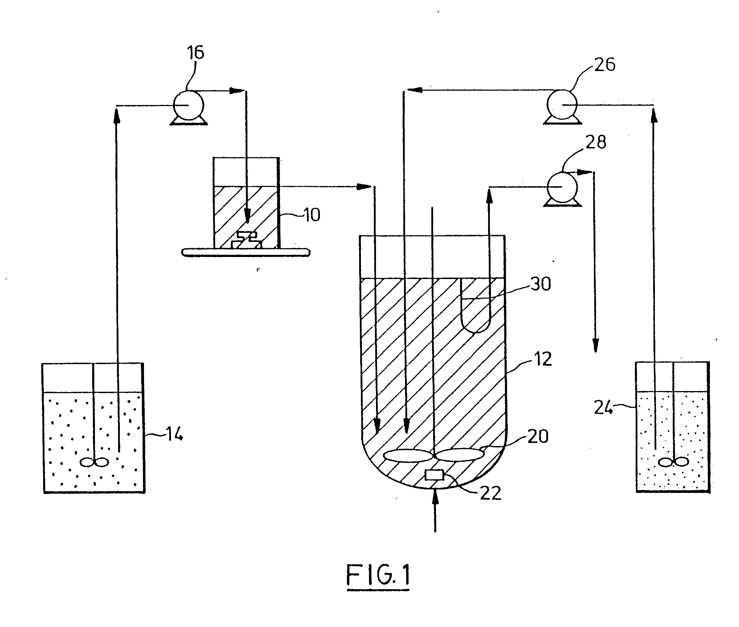

A schematic representation of the two-stage continuous process is shown in Figure 1. First and second constant volume fermentors 10 and 12 are operated in tandem. A mineral salts culture medium is pumped from a reservoir 14 through pump 16 at a constant rate to the first fermentor 10. The culture medium includes assimilable carbon and nitrogen with other nutrients and inorganic salts required by the microorganism for its growth. Inorganic nitrogen is included in a growth limiting amount, while all other essential growth elements are included in excess. The first fermentor 10 is typically a state-of-the art chemostat which is operated at about 30°C, pH 7 with thorough mixing and aeration. For example, a fermentor with a working volume of 340 ml can be operated with agitation of 600 rpm and an aeration rate of 470 cc/min. The first fermentor 10 is operated at a dilution rate so as to achieve maximum biomass productivity with almost negligible inorganic nitrogen in the effluent from the fermentor.

The effluent from the first fermentor 10 is fed directly to the second stage fermentor 12, where the biomass synthesizes the desired exopolysaccharide. Figure 1 illustrates an exemplary type of fermentor which can be operated with moderate agitation which does not significantly decrease the intrinsic viscosity of the exopolysaccharide being produced. As shown, the bottom of the fermentor is rounded, and agitation is achieved with a single marine-type stainless steel (SS) propeller 20, with air being introduced through a sintered-glass disk sparger 22 located directly under the rotating propeller for good gas dispersion and oxygen transfer.

A nitrogen-free sterile glucose solution, from which the microorganism can synthesize the desired exopolysaccharide product is fed continuously from stirred holding tank 24, through pump 26. Alternatively excess glucose can be added to the first fermentor 10, which can spill over to the second fermentor 12. A continuously operating pump 28, coupled to an exit weir 30 maintains a constant volume in the second fermentor 12. The volume and dilution rate for the second fermentor are fixed such that the residence time of the organism in the second stage is equivalent to the maximum length of time during which the activity of a batch culture with respect to product synthesis is maximal. Glucose is added at a rate which does not exceed the rate at which it can be utilized for exopolysaccharide synthesis. The pH of the second stage is most preferably about 5.9 and the temperature about 30°C.

Product recovery from the effluent of the second fermentor 12 can be performed by known methods (see H. Harada et al., J. Ferment. Technol. 46, 679-684, 1968). The exopolysaccharide is extracellular in its location, but it is usually associated with the cell surface in such a manner that usual separation techniques for removing insoluble materials, such as centrifugation and filtration would remove both the bacterial cell mass and the desired exopolysaccharide product. The exopolysaccharide product can be effectively dissociated from the cell surface and solubilized by treatment with alkali to allow removal of the cells and is then isolated from the fermentation broth after neutralizing with acid. The isolated exopolysaccharide product is washed to remove any salts associated therewith. Salts can affect the intrinsic viscosity measurements of the product. Generally, water washing several times with distilled or deionized water is performed. Alternate known isolation techniques are described by Kimura et al. in U.S. Patent 3,754,925.

The isolated β-1,3-glucan exopolysaccharide can be preserved by dehydration using commonly known procedures including spray drying, lyophilization and dehydration with non-polar organic solvents such as acetone or isopropyl alcohol.

The β-1,3-glucan exopolysaccharide product of this invention has an intrinsic viscosity of at least about 6.0 dL/g. It should be noted that this product is generally isolated after an elapsed fermentation time (EFT) of at least about 48 hours, in order to have a high and economic yield of the product. In a batch process, this 48 hour EFT is measured from the onset of the stationary phase. However, in a continuous process, the 48 hour EFT refers to a residence time in the stationary phase of at least about 48 hours.

The physicochemical properties of the novel β-1,3-glucan exopolysaccharide product, which are notably distinct from those of known samples of curdlan-type products, particularly from the Wako curdlan product, are set forth in Table 2. The measurements are carried out in accordance with the procedures to follow.

Table 2

| Micro-Organism | Intrinsic Viscosity (dL/g) | Gel Strength (g/10.2cm²) | Molecular Weight (daltons x 10⁶)* |
|---|---|---|---|
| A. faecalis var. mxyogenes ATCC 31749 | 9.0 | >1200 | 2.3 |
| A. faecalis var. mxyogenes ATCC 21680 | 7.7 | 750 | 1.9 |
| Agrobacterium radiobacter ATCC 21679 | 6.4 | >1200 | 2.3 |

\* Minimum values, higher average values were obtained

The gels prepared from the novel product, as described hereinbelow have deformation measurements >70% and compressibility/rigidity values >50 g/mm.

For comparison purposes, the physicochemical properties for the Wako curdlan product, using the same techniques set out hereinbelow were as follows:

| | |
|---|---|
| Intrinsic viscosity - | 4.0 - 5.0 dL/g |
| Gel Strength - | 500 - 750 g/10.2 cm² |
| Molecular weight - | 0.8 - 1.2 x 10⁶ daltons |
| Compressibility - | < 50 g/mm |
| Deformation - | < 70% |

It is difficult to compare these measured values for the Wako curdlan product with reported literature values for previous curdlan-like products, since different techniques and conditions affect the measured values. Ogawa et al., Carbohydrate Res. 23, 399, 1972, noted an intrinsic viscosity for PS-13140 (product of A. faecalis var. myxogenes strain IFO 13140, ATCC 21680) using a Ubbelohde-type dilution viscometer at 30°C and concentrations of $OH^- > 0.25N$ of about 3.5 dL/g. The gel strength techniques of Kimura et al. in U.S. Patent 3,754,925 are significantly different from the techniques described herein, making the values difficult to compare. Some comparison of molecular weight values can be made. Harada et al., Arch. Biochem. Biophys., 124, 292, 1968, reported a degree of polymerization, DPn, of 135, which, from a value of 162 for the molecular weight of the glucose units, corresponds to a molecular weight of 21,870 daltons. Similar calculations from data from Saito et al., Agr. Biol. Chem., 32, 1261, 1968, yield molecular weight values of 21,870 to 73,710 daltones. Kimura et al., U.S. Patent 3,754,925 give molecular weight values of 40,500 to 79,380 daltons from their reported DPn values.

Further comparative physicochemical characteristics are set out below in Table 2A

8

EP 0 322 393 A1

## TABLE 2A

## COMPARATIVE PHYSICOCHEMICAL CHARACTERISTICS

| TEST | WAKO PRODUCT | PRODUCT OF INVENTION |
|---|---|---|
| Infrared Spectrum | Similar IR Spectra obtained; significant absorption bands found at the following wavenumbers $(cm^{-1})$ 3600-3200, 2950-2900, 1640, 1420, 1365, 1310, 1260, 1200, 1160, 1120, 1100, 1080, 1070, 1040, 1020, 980, 890. | |
| Specific Rotation [$\alpha$] $D^{25}$ | $+17° \pm 5°$ | $+20° \pm 5°$.* |
| UV Spectrum | Essentially no UV absorption | |
| Solubility Water (pH=7) DMSO Ethanol | No Yes No | No Yes No |
| $C^{13}$ Nuclear Magnetic Resonance (N.M.R.) Spectra (in DMSO, ppm) | Similar spectra obtained with significant absorption at ($\delta$,ppm): 87 (Cl), 77 (C3), 73 (C5), 56 (C2) 39.5 (C4), 22.6 (C6) | |

* Values for specific rotation were found to vary considerably between samples. This value was measured for product produced by ATCC 21680. However, a cloudy sample from ATCC 31749 gave a value of +29°. U.S. Patent 3,754,925 reports a value of +33° ± 6° for PS-13140, while Harada et al. report a value of +18°.

Since the values given for the above measurements can vary considerably unless consistent techniques are used, the techniques used for the measurements are described in detail below.

The high gel strength, rigidity and compressibility values for the product of this invention are of considerable economic importance, since less of the product would be required to achieve a specified gel strength in a composition containing this product. The higher gel strength and rigidity values should also result in improved, stronger film characteristics in film products produced from the exopolysaccharide. A further advantage is associated with the high intrinsic viscosity values, namely improved consistency. Since no significant decrease in intrinsic viscosity occurs, it is possible to isolate an exopolysaccharide product having reproducible physiocochemical properties.

Intrinsic viscosity measurements are performed on dilute solutions of a salt free (water washed several times) sample of the exopolysaccharide in the range of 0.02 to 0.05% (dry wt/vol) in 0.3N NaOH at 25°C (+/-0.1°C). The apparatus used is a Ubbelohde-type capillary viscometer, designed by Prof. J. Guillet of University of Toronto, described by Kilp et al., Rev. Sci. Instrum. 47, 1496, 1976 and is manufactured by Ecoplastics Ltd. of Willowdale, Ontario, Canada. The apparatus determines the increase in viscosity of the

9

product in solution attributed solely to the polymer (within 0.05% error).

Molecular weight determinations are performed by gel permeation size exclusion chromatography (GPC). The apparatus consists of two Spherogel TSK columns 17.5 mm ID, (Bio-Rad Lab Ltd., Mississauga, Ontario, Canada) in series with lengths of 30 and 60 cm respectively. Product samples (0.05% in 0.1N NaOH, 40°C) are pumped (Beckman model 110A solvent pump) through the columns at a constant rate of 1 ml/min and detected using a Waters Model 410 differential refractometer (Waters, Milford, U.S.A.). Chromatograms are characterized by a Spectra Physics data processing system (Bio-Rad Lab Ltd., Mississauga, Ontario, Canada). Polymer standards are polystyrene derivatives (Waters, Milford, MA and Pressure Chemical Co., Pittsburgh, PA) with molecular weights ranging from 16,000 to 1,060,000 daltons. Calibration consists of determining the relationship between elution time and the log of the molecular weight.

Gel strength measurements are performed after performing standardized homogenization and gelation steps with the product. A 1.5% suspension of the product in water is prepared and homogenized with a Polytron 5 mm probe (Brinkman) at the number 5 setting for 3 min. A 5 ml aliquot of this suspension is transferred to a pyrex test tube (ID 14mm) and vortexed for 15 seconds. The test tube is immersed in a water bath at 95°C for 10 min to form the thermo-gel and cooled in a cool water bath for 10 minutes. The product is then removed from the test tube can cut into 10 mm wide samples along the length of the gel. The gel samples are then compressed using the Instron Compression Tester and an anvil with a 36 mm diameter. The testing parameters for gel strength are as follows:

| | |
|---|---|
| Gauge Length (mm) | 11.0 |
| Cross Head Speed (mm/min) | 20.0 |
| Chart Speed (mm/min) | 200.0 |
| Cycle at (mm) | 9.0 |

Gel strength is measured ($g/10.2\ cm^2$) as the force required to rupture the gel during compression.

Deformation (%) and compressibility or rigidity (g/mm) are measured on the above-described gel. For deformation, from the gel strength Instron chart, the extent to which the gel is deformed at the point of rupture is calculated. From the gel strength Instron chart compressibility/rigidity is calculated from the slope of the rising portion of the curve.

The invention is further illustrated in the following non-limiting examples.

## Example 1

This example is included to illustrate the combined effect of both aeration and agitation, in accordance with the invention, on firstly the "quality" of the exopolymer produced by the bacterial culture (as determined by the intrinsic viscosity of isolated β-1,3-glucan exopolysaccharide product as a function of elapsed fermentation time, EFT) and secondly on the "quantity" of β-1,3-glucan exopolymer produced as a function of EFT (i.e. the specific rate of β-1,3-glucan production, $q_p$).

One fermentation used a conventional baffled turbine-agitated bioreactor, similar to those used in prior art techniques of producing curdlan-type products. A second fermentation process used the above reactor, after replacing two of the uppermost turbine impellers with a single marine-type propeller. A third fermentation, in accordance with the invention, used a rounded bottom fermentor stirred by a single marine-type propeller.

The modified defined mineral salts medium (MSM) of Phillips et al., (Can. J. Microbiol. 29, 1331, 1983) was used in all fermentations and contained the following: $KH_2PO_4$ (12.8mM); $K_2HPO_4$ (2.8mM); $Na_2SO_4$ (2.9mM); $FeCl_3.6H_2O$ (90μM); $CaCl_2.2H_2O$ (100μM); $MgCl_2.6H_2O$ (1.25mM); $MnCl_2.4H_2O$ (50μM); citric acid (1.0mM) and polypropylene glycol 2025 (0.1ml/L) as antifoam. Since the growth yield coefficient ($Y_N$) with respect to assimilable nitrogen (where the sole nitrogen source for growth is ammonium chloride) is known to be about 2 g DW cells/g $NH_4Cl$ (Phillips et al., Can. J. Microbiol. 29, 1331, 1983), the desired level of biomass was determined by the amount of added $NH_4Cl$ usually being 28mM and yielding a cell density (dry weight basis) of about 3 g/L. The carbon source was D-glucose (50 g/L) and was sterilized separately. Glass distilled water was used and sterilization of the fermentation medium was accomplished by autoclaving at 121°C.

A batch fermentation with Alcaligenes faecalis ATCC 31749 was conducted in a stirred tank bioreactor of standard or conventional design (MultiGen model F2000 manufactured by New Brunswick Scientific Co., Edison, NJ, USA) with agitation/mixing being accomplished by a rotating central shaft to which were affixed multiple (3) Rushton-type flat-blade turbine impellers (diam. = 4.9 cm) placed equidistantly within the liquid volume. The flat bottom glass reactor vessel (diam. = 11 cm) was baffled with three flat blades fixed into the rubber headplate and had a working volume of approximately 1500 ml. The agitation speed was controlled at 600 RPM during the growth phase and increased to 850 RPM during the polymer production phase of the fermentation. The culture was sparged with air, exiting through small holes located at the bottom of the hollow central shaft, at a constant rate of 500 cc/min equivalent to 0.33 V/V/M.

The pH was monitored by means of an Ingold combination (in-place sterilizable) electrode and controlled (NBS model pH40 Controller) at a constant pH value by means of the addition of titrant (2N KOH). The pH was kept constant at 7.0 during growth and adjusted to 5.9 (with 1N HCl using the pH controller) at the onset of stationary-phase (i.e. when growth ceased). The temperature was maintained at 30°C throughout the fermentation.

The second fermentation was conducted under the above conditions, but the bioreactor was modified by replacing two of the uppermost three flat-blade turbine impellers with a marine propeller (diam. = 4.6 cm). The shaft was rotated at constant speed of 750 RPM throughout the entire course of the fermentation.

In the third fermentation, in accordance with one embodiment of this invention, A. faecalis ATCC 31749 was cultured as above except in a modified NBS MultiGen bench-top fermentor (model F2000). The bottom of the glass vessel (diam. = 11 cm) was rounded and agitation was accomplished by means of a stainless steel (SS) shaft penetrating into the vessel through a sealed bearing fixed in the rubber headplate to accommodate agitation/mixing by an overhead variable-speed motor drive. A single marine-type SS propeller (Cole Parmer Scientific Co., Chicago, USA) with a diameter of 7.0 cm was fixed to the bottom of the solid SS shaft and placed about 4.0 cm above the bottom of the vessel. The propeller was rotated at a fixed speed of 600 RPM during the growth phase but was reduced to 500 RPM after growth ceased. The culture was ventilated with filtered air introduced at a rate of 500 cc/min. (0.33 V/V/M) through a sintered-glass disk sparger (diam. = 30 mm, pore size 10-15 um) located directly under the rotating propeller for good gas dispersion and $O_2$ transfer.

To recover the exopolysaccharide product from the fermentation broth, equal volumes of the fermentation broth and 0.6N sodium hydroxide were added together and stirred for 20-30 min. The bacterial cell mass was removed from suspension by centrifugation at about 10,000 x g for 10-15 min. The cell-free supernatant was decanted and neutralized to pH 7 (with constant stirring) with 4N acetic acid. The exopolysaccharide formed a loose water-insoluble gel at pH 7 and was removed from suspension by centrifugation at about 10,000 x g for 10-15 min. The gel pellet was washed thoroughly (usually three times) by repeated resuspension/centrifugation to remove the salt formed during the recovery procedure. The isolated salt-free gel was preserved at 4°C as a wet gel. Alternatively, the gel was preserved in a dry state by several dehydration procedures known to those skilled in the art, such as acetone dehydration, lyophilization or spray drying.

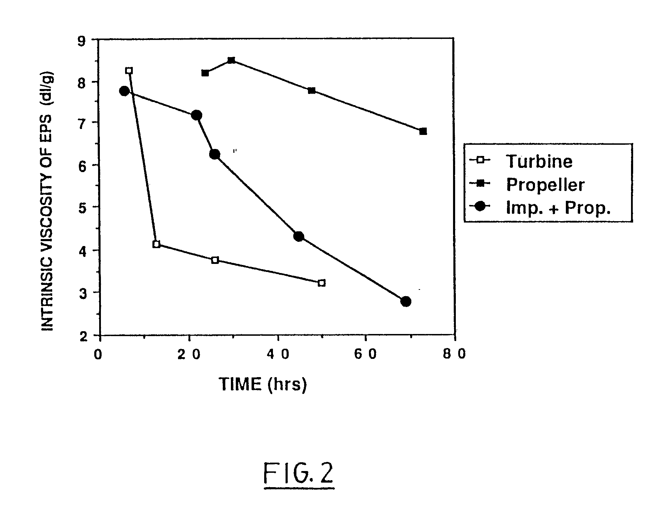

In each of the three fermentation processes, samples of the fermentation broth were removed from the bioreactor at several elapsed fermentation times (time zero being measured from the commencement of the stationary-phase when growth ceased) and processed as described above in order to recover the β-1,3-glucan exopolymer. The intrinsic viscosity of each sample was measured. The results are shown in Figure 2. In each of the fermentation trials the OTR was determined by the standard chemical method using sulphite oxidation with cupric ions as catalyst (C.M. Cooper et al., Ind. Eng. Chem. 36, 504-509, 1944). The effect of changes in the bioreactor design, with respect to agitation and aeration, (i) on the specific rate of exopolymer production ($q_p$), and (ii) on the "quality" of the recovered exopolymer as judged by the intrinsic viscosity and molecular weight (MW) are summarized in Table 3, includes at the end of the examples.

It is evident that the fermentations conducted with the Rushton-type, flat-bladed turbine impellers, even if retrofitted with a single marine-type propeller in place of the uppermost turbine impellers, produced an exopolysaccharide product, the intrinsic viscosity of which decreased significantly with elapsed fermentation time (Fig. 2; also Table 3, Runs No. 1 & 2). A gel strength measurement on the product from Run No. 1 gave a value of only 250 g/10.Zcm². It is also apparent from these two fermentation trials that such an alteration in bioreactor design with respect to agitation had a pronounced effect on the OTR which was reflected in a decrease in the specific rate of exopolymer production ($q_p$) (Table 3, Runs No. 1 & 2). The use of the marine-type propeller in the third fermentation produced a product of high intrinsic viscosity which did not decrease significantly with elapsed fermentation time (EFT) (Fig. 2). After 72 hours EFT, the intrinsic viscosity of the isolated product had dropped only to about 7.0 dL/g, from a value of about 8.5 dL/g at about 32 hours EFT. In the fermentation runs utilizing the single larger diameter marine-type propeller, at reduced speed (500 RPM), the air was introduced through a sintered-glass disk located underneath the propeller. Although this configuration, with respect to agitation and aeration, lead to a decrease in OTR compared to the standard design with turbine impellers at comparable rates of air sparging (0.33 V/V/M), the $q_p$ was relatively unaffected. However, when the air sparging rate was reduced from 0.33 V/V/M to either 0.15 or 0.08 V/V/M, the OTR was reduced to a level which negatively affected the $q_p$ (Table, 3, Runs No. 3 & 4).

The procedure used for the first fermentation (Table 3, Run No. 1) with conventional Rushton-type turbine impellers was repeated, using slower agitation speeds. Lowering the agitation speed to 600 RPM in the stationary phase did not result in an increased intrinsic viscosity of the isolated exopolymer; in fact, the intrinsic viscosity after 48 hours was found to be 3.2 dL/g, compared to an intrinsic viscosity of 3.5 dL/g after 48 hours EFT when the impeller speed had been 850 RPM.

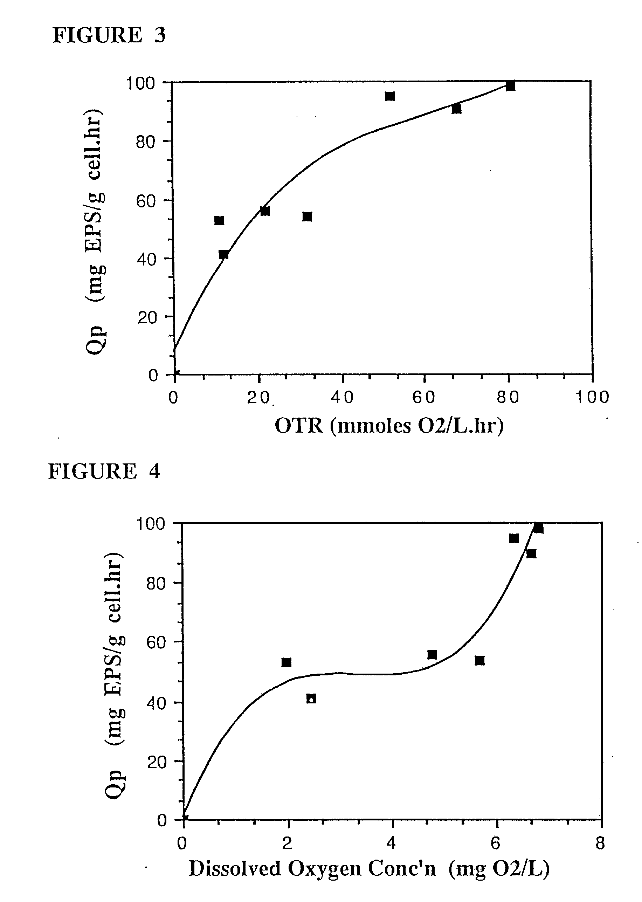

The effect of OTR and the equivalent DO on $q_p$ (for A. faecalis ATCC 31749) is summarized in Figures 3 and 4 . respectively. It will be noted at a DO value of 2.0 mg $O_2$/L or greater, $q_p$ was increased over what it would be had the DO been maintained at a value just sufficient for growth requirements, say 1.0 mg $O_2$/L. In general, a DO value of 6.0 mg $O_2$/L or greater can be seen to be preferred. To maximize $q_p$, a DO value of 6.8 mg $O_2$/L is preferred.

In the following Examples 2-5, β-1,3-glucan exopolysaccharide products, having intrinsic viscosities greater than 6.0 dL/g, were produced with different bioreactors that were variously configured with respect to both agitation/mixing and aeration. The products were isolated from the fermentation broth using the procedure set out in Example 1. The physicochemical properties were measured as above-described.

Example 2

This example is included to demonstrate the operability of the process of this invention with other strains of -1,3-glucan exopolysaccharide producing microorganisms. The fermentation was conducted in accordance with the procedure set forth in Example 1, in the round bottom bioreactor equipped with a single marine-type propeller (diam = 7 cm). The microorganisms cultured included Alcaligenes faecalis ATCC 31749 and ATCC 21680 (IFO 13140) and Agrobacterium radiobacter ATCC 21679 (IFO 13126). A. faecalis ATCC 21680 is an auxotrophic mutant requiring uracil for growth and this nutrient was added to the culture medium at 0.1 g/L (sterilized separately). Two speeds of agitation were used, 500 and 600 RPM during the stationary phase.

Samples of the fermentation broth were withdrawn and $\beta$-1,3-glucan exopolymer isolated after 48 hours EFT. For strain ATCC 21680, another sample was withdrawn and isolated after 72 hours EFT to demonstrate that the intrinsic viscosity of the exopolysaccharide product had not significantly decreased.

The measured parameters, OTR, $q_p$, intrinsic viscosities and MW are summarized in Table 3 as Run Nos. 5 & 6 for strain ATCC 31749 at 500 and 600 RPM respectively, Run No. 12 for strain ATCC 21680 and Run No. 13 for strain ATCC 21679. A gel strength measurement on the product from Run 5 gave a value greater than 1200 g/10.2cm$^2$.

It will be noted that the values of intrinsic viscosities are significantly higher than for the products which were isolated after similar EFT with a bioreactor using turbine impellers, and in all cases were greater than 6.0 dL/g.

As in Example 1, the effect of decreased aeration was examined using strain ATCC 21680. It will be noted that this strain behaved in a similar manner to strain 31749 with respect to a decreased value for $q_p$ at the lower rates of air sparging (Table 3, Runs No. 10 & 11).

Example 3

This example demonstrates that a -1,3-glucan exopolysaccharide of high viscosity can be produced in a bioreactor equipped with a helical propeller. A. faecalis ATCC 31749 was cultured as in Example 1, in a modified NBS MultiGen F2000 bench-top fermentor except the marine-type propeller was replaced by a 3-blade helical propeller (diam. = 5.5 cm) purchased from Biolafitte Ltd. (Poissy, France). The propeller was rotated at 600 RPM during growth phase but the speed was reduced to 500 RPM at the onset of stationary phase after growth ceased. The culture was ventilated with filtered air introduced at 0.33 V/V/M through a sintered glass disk sparger as described in Example 1.

Under these operating conditions the OTR was 68 mmoles $O_2$/L.hr and the $q_p$ was near the maximum observed in these trials. The product sample which was isolated after 48 hours EFT had an intrinsic viscosity of 9.7 dL/g (see Table 3, Run No. 7).

Example 4

A high intrinsic viscosity product of this invention was also produced using a bioreactor fitted with a draught tube and variable pitch propellers. A. faecalis ATCC 31749 was cultured in a mineral salts medium in a model E, 15L bioreactor manufactured by B. Braun (Billingcame, CA, USA) fitted with a draught tube and three (3) variable pitch (set at 30°) propellers (diam = 8.9 cm). The working volume was 10L. The propellers were rotated at a fixed speed of 600 RPM during the growth phase but the speed was reduced to 500 RPM during the stationary phase. The culture was ventilated with filtered air introduced through a ring sparger, located beneath the bottom propeller, at a constant rate of 0.35 V/V/M.

Under these operation conditions, the OTR was only 12 mmoles $O_2$/L.hr, which was insufficient to support a high rate of polymer production ($q_p$ = 41 mg exopolymer/g cell.hr). Although the quantity of polymer produced was less than ideal, the quality of the product was improved over that produced in the conventional bioreactor with turbine impellers. The intrinsic viscosity of the exopolymer isolated after 48 hours EFT was 7.9 dL/g (Table 3, Run No. 8).

This example illustrates that low oxygen tension affects primarily the rate of exopolymer production (quantity) and not the "quality" of the polymer.

Example 5

This example demonstrates that the process described in this invention can be scaled up to a volume of 200 litres, to obtain a high quality product. The fermentation was conducted with A. faecalis ATCC 31749 in an insulated New Brunswick Scientific Model IF250 fermentor ($D_t$ = 53.3cm, D /$D_t$ = 0.57), equipped with two hydrofoil impellers of 30.5 cm diameter (Prochem Mixing Equipment Ltd., Brampton, Ontario). The agitation speed was 180 RPM, which gives a tip velocity of about 170 m/min, which is comparable to the tip velocity of a 7 cm propeller at an agitation speed of about 770 RPM. The culture was sparged with air through a microporous stainless steel filter (Pall Canada Ltd.) at a constant rate of 100 L/min, equivalent to 0.5 V/V/M. The pH was controlled at 7.0 during growth and adjusted to 5.9 for the stationary phase. The temperature was maintained at 30°C throughout the fermentation.

The OTR under these conditions was measured to be 42 mmoles $O_2$/L.hr; the calculated dissolved oxygen level would by 6.1 mg $O_2$/L. The specific rate of polymer production ($q_p$) was 77 mg/g cell/hr, which is close to the values which would be extrapolated from Figures 3 and 4 for such OTR and DO values based on fermentations of smaller scale.

The product isolated over the course of the run was found to have high intrinsic viscosity, gel strength and MW. At 24 hours EFT, the product had a viscosity of 9.5 dL/g and a gel strength of 2400 g/10.2 cm$^2$. The

properties of the product at 48 hours EFT are shown in Table 3, Run No. 9.

## TABLE 3  EFFECT OF BIOREACTOR DESIGN WITH RESPECT TO AERATION & AGITATION ON PRODUCT QUALITY AND SPECIFIC RATE OF EXOPOLYMER PRODUCTION

| FERMENTOR DESIGN and CONFIGURATION | OPERATIONAL PARAMETERS | | | | | | | |
|---|---|---|---|---|---|---|---|---|
| Run No. | Liquid Vol. (ml) | $D_i/D_t$ | Air Flow Rate (V/V/M) | Rot. Speed (RPM) | OTR mmoles $O_2$/L·hr | $q_p$ mg/g cell/hr | Intrinsic Visc. (48hrs) dL/g | MW x $10^6$ (48hrs) |
| *Alcaligenes faecalis* ATCC 31749 | | | | | | | | |
| Air sparged through holes at bottom of central shaft | | | | | | | | |
| 1. NBS MultiGen model F2000 3 turbine impellers Diam. = 4.9 cm | 1500 | 0.45 | 0.33 | 850 | 80 | 98 | 3.5 | 0.5 |
| 2. MultiGen F2000 1 prop (4.6 cm) + 1 imp (4.9 cm) | 1500 | 0.45 | 0.33 | 750 | 32 | 54 | 4.3 | – |
| Air sparged through sintered glass disk (3 cm) under propeller | | | | | | | | |
| 3. Round bottom tank Single prop (7 cm) | 1500 | 0.64 | 0.08 | 500 | 11 | 53 | 5.8 | – |
| 4. Round bottom tank Single prop (7 cm) | 1500 | 0.64 | 0.15 | 500 | 22 | 56 | 6.3 | – |
| 5. Round bottom tank Single prop (7 cm) | 1500 | 0.64 | 0.33 | 500 | 52 | 95 | 9.0 | 2.3 |
| 6. Round bottom tank Single prop (7 cm) | 1500 | 0.64 | 0.33 | 600 | 64 | 108 | 7.3 | – |
| Air sparged through sintered glass disk (3 cm) under propeller | | | | | | | | |
| 7. Round bottom tank Helical prop (5.5 cm) | 1500 | 0.50 | 0.33 | 500 | 68 | 90 | 9.7 | – |
| Air through ring sparger under bottom propeller | | | | | | | | |
| 8. B.Braun (model E) draught tube & 3 var. pitch props. | 10,000 | 0.45 | 0.35 | 500 | 12 | 41 | 7.9 | – |
| Air through microporous SS sparger under bottom hydrofoil impeller | | | | | | | | |
| 9. NBS (model IF250) 2 Hydrofoil impellers (30.5cm) | 200 L | 0.57 | 0.50 | 180 | 42 | 77 | 7.7 | 2.3 |

13

EP 0 322 393 A1

TABLE 3 (continued)

| FERMENTOR DESIGN and CONFIGURATION | | | | OPERATIONAL PARAMETERS | | | | |
|---|---|---|---|---|---|---|---|---|
| Run No. | Liquid Vol. (ml) | $D_i/D_t$ | Air Flow Rate (V/V/M) | Rot. Speed (RPM) | OTR mmoles $O_2$/L·hr | $q_p$ mg/g cell/hr | Intrinsic Visc. (48hrs) dL/g | MW x $10^6$ (48hrs) |

*Alcaligenes faecalis* ATCC 21680

Air sparged through sintered glass disk (3 cm) under propeller

| | | | | | | | | |
|---|---|---|---|---|---|---|---|---|
| 10. Round bottom tank Single prop (7 cm) | 1500 | 0.64 | 0.08 | 500 | 11 | 69 | 6.9 | - |
| 11. Round bottom tank Single prop (7 cm) | 1500 | 0.64 | 0.15 | 500 | 22 | 74 | - | - |
| 12. Round bottom tank Single prop (7 cm) | 1500 | 0.64 | 0.33 | 500 | 52 | 102 | 7.7 | 1.9 |

*Agrobacterium radiobacter* ATCC 21679

Air sparged through sintered glass disk (3 cm) under propeller

| | | | | | | | | |
|---|---|---|---|---|---|---|---|---|
| 13. Round bottom tank Single prop (7 cm) | 1500 | 0.64 | 0.33 | 500 | 52 | 89 | 8.0 | 2.3 |

The $D_i$ = diam. of agitation device, either Rushton turbine impeller, helical, marine-type propeller or hydrofoil impeller; $D_t$ = diam. of fermentation tank. The NBS MultiGen F2000 glass fermentor is a magnetically-coupled bottom drive unit.The modified F2000 (round bottom) was fitted with a variable speed, top-drive motor. "-" indicates not determined.

## Claims

1. An improved, water insoluble, gel-forming β-1,3-glucan exopolysaccharide consisting of linearly connected D-glucose residues attached through β-1,3 linkages and having one or more of the following characteristics, an intrinsic viscosity, as measured by the procedure described in the disclosure, of at least about 6.0 dL/g, a molecular weight, as measured by the gel permeation size exclusion chromatography procedure described in the disclosure, of at least about 1.9 x $10^6$, and a gel strength, as measured by the procedure described in the disclosure, of at least about 750 g/10.2 cm$^2$.

2. The β-1,3-glucan exopolysaccharide as set forth in claim 1, having a gel strength, as measured by the procedure described in the disclosure, of at least about 1,000 g/10.2 cm$^2$, a molecular weight, as measured by the gel permeation size exclusion chromatography procedure described in the disclosure, of at least about 2.0 x $10^6$, and an intrinsic viscosity, as measured by the procedure described in the disclosure, after at least 48 hours elapsed fermentation time of at least about 6.0 dL/g.

3. A process for producing the improved, water insoluble gel-forming β-1,3-glucan exopolysaccharide, as defined in claim 1, comprising:

(a) cultivating a β-1,3-glucan exopolysaccharide producing strain of microorganism in two phases, a growth phase and a non-growth stationary exopolysaccharide biosynthesis phase, the growth

14

phase being conducted in an aerobic, agitated aqueous medium containing assimilable carbon, nitrogen and inorganic salts and any other essential growth factors, the amount of nitrogen being limited so that at the end of the growth phase the medium contains substantially no assimilable nitrogen, at a temperature between about 27 and 33°C and at a pH between about 5 and 8, the non-growth phase being conducted in an aerobic agitated aqueous medium containing assimilable carbon but substantially no assimilable nitrogen, at a temperature between about 27 and 33°C and a pH between about 5 and 8, wherein agitation in the stationary phase provides mixing sufficient to produce a homogeneous medium and oxygen transfer sufficient for the aerobic biosynthesis of the exopolysaccharide product, without significantly decreasing the intrinsic viscosity of the exopolysaccharide product which is produced below about 6.0 dL/g;

(b) solubilizing the exopolysaccharide product so produced, by treatment with alkali and removing the cell mass; and

(c) isolating the exopolysaccharide from the fermentation broth.

4. The process as set forth in claim 3, wherein agitation is accomplished in a bioreactor with a marine-type propeller which may be equipped with a draught tube to facilitate aeration and mixing, or wherein the agitation is accomplished in a bioreactor with a helical-type propeller or a hydrofoil impeller.

5. The process as set forth in claim 3, wherein the process is conducted as a two-stage continuous process, the growth phase being conducted in a first fermentor, the effluent from which is introduced into a second fermentor for the stationary exopolysaccharide biosynthesis phase, and wherein the pH in the first fermentor is between about 6.8 and 7.2 and the pH in the second fermentor is between about 5.7 and 6.2.

6. The process as set forth in claim 3, 4 or 5, wherein, in the stationary phase, the dissolved oxygen concentration in the aqueous medium is maintained at a value of at least about 2.0 mg $O_2$/L in order to increase the specific rate of exopolysaccharide production ($q_p$).

7. The process as set forth in claim 5, wherein, in the stationary phase, the dissolved oxygen concentration in the aqueous medium is maintained at a value of at least about 6.0 mg $O_2$/L in order to increase the specific rate of exopolysaccharide production ($q_p$).

8. The process as set forth in claim 5, wherein, in the stationary phase, the dissolved oxygen concentration in the aqueous medium is maintained at a value of at least about 6.8 mg $O_2$/L in order to increase the specific rate of exopolysaccharide product production ($q_p$).

9. The process as set forth in claim 3, 7 or 8, wherein the microorganism is of the genus Alcaligenes or Agrobacterium.

10. The process as set forth in claim 3, 7 or 8, wherein the microorganism is selected from A. faecalis ATCC 31749, A. faecalis ATCC 31750, A. faecalis ATCC 21680 and Agrobacterium radiobacter ATCC 21679.

FIG.1

FIG. 2

## FIGURE 3

Qp (mg EPS/g cell.hr) vs OTR (mmoles O2/L.hr)

## FIGURE 4

Qp (mg EPS/g cell.hr) vs Dissolved Oxygen Conc'n (mg O2/L)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | PATENT ABSTRACTS OF JAPAN, vol. 7, no. 252 (C-194)[1397], 9th November 1983, page 73 C 194; & JP-A-58 138 392 (NIPPON GOSEI GOMU K.K.) 17-08-1983 * Abstract * | 1 | C 12 P 19/04 |
| A | FR-A-2 406 447 (M. SUGIURA) * Page 2, lines 1-7; example 1 * | 1 | |
| A | GB-A-2 090 847 (GEORGE WESTON LTD) * Claim 1; example 1 * & US-A-4 355 106 (Cat. D) | 1,3 | |
| A | GB-A-2 094 324 (TAKARA SHUZO CO.) * Page 2, lines 30-45; page 5, lines 51,52; example 2 * | 1,3 | |
| D,Y | BIOTECHNOLOGY LETTERS, vol. 8, no. 3, March 1986, pages 145-150, Kew, GB; H. LAWFORD et al.: "Influence of bioreactor design on the rate and amount of curdlan-type exopolysaccharide production by Alcaligenes faecalis" * Abstract; tables 3,4 * | 3,4 | |
| Y | CHEMICAL ABSTRACTS, vol. 106, no. 3, 19th January 1987, page 475, abstract no. 17012k, Columbus, Ohio, US; K. GBEWONYO et al.: "The use of hydrofoil impellers to improve oxygen transfer efficiency in viscous mycelial fermentations", & BIOREACT. FLUID DYN., PAP. INT. CONF. 1986, 281-99 * Abstract * -/- | 3,4 | |

TECHNICAL FIELDS
SEARCHED (Int. Cl.4)

C 12 P
C 08 B
C 12 M

The present search report has been drawn up for all claims

| Place of search | Date of completion of the search | Examiner |
|---|---|---|
| THE HAGUE | 03-04-1989 | SOMERVILLE F.M. |

EPO FORM 1503 03.82 (P0401)

European Patent
Office

**EUROPEAN SEARCH REPORT**

Application Number

EP 88 85 0435

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 93, no. 1, 7th July 1980, page 580, abstract no. 6117k, Columbus, Ohio, US; A. EINSELE et al.: "Design and characterization of a completely filled stirred bioreactor", & EUR. J. APPL. MICROBIOL. BIOTECHNOL. 1980, 9(2), 83-91 * Abstract * | 3,4 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 03-04-1989 | SOMERVILLE F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)